# EUROPEAN PATENT APPLICATION

(11) **EP 3 915 611 A1**
(43) Date of publication of application: **01.12.2021**
(21) Application number: 19918861.6
(22) Date of filing: 25.12.2019
(51) Int. Cl.: A61M 5/142

(54) **MEDICAL FLUID ADMINISTRATION APPARATUS AND CRADLE DEVICE**

(30) Priority: 13.03.2019 JP 2019045413
(71) Applicant: TERUMO Kabushiki Kaisha, Tokyo 151-0072 (JP)
(72) Inventor: UCHIYAMA Joji, Ashigarakami-gun, Kanagawa 259-0151 (JP); YAMAZAKI Tsuyoshi, Ashigarakami-gun, Kanagawa 259-0151 (JP)
(74) Representative: Casalonga
(86) International application number: PCT/JP2019/050929
(87) International publication number: WO 2020/183866

(57) **Abstract**

A drug solution administration apparatus includes a pump main body, a cradle device, and a connection port. The cradle device has side wall portions that rise in a third direction Z from both end portions in a second direction on a placement surface portion, and face a side surface portion. The side wall portion has a posture correction section that is attached to or faces a corner portion where an upper surface portion and a side surface portion are connected to each other in a housing.

## Description

### Technical Field

The present invention relates to a drug solution administration apparatus, for example, a drug solution administration apparatus for performing continuous drug solution administration such as an insulin pump, and a cradle device capable of supporting a pump main body.

### Background Art

In recent years, treatments in which drug solution is continuously administered into the body of a patient is performed by subcutaneous injection or intravenous injection. For example, as a treatment for diabetic patients, a continuous injection of a trace amount of insulin into the body of a patient has been implemented. In this treatment, a portable drug solution administration apparatus (so-called insulin pump) that can be fixed to the body or clothing of a patient and carried around is used to administer the drug solution (insulin) to the patient throughout the day.

In such a portable drug solution administration apparatus, firstly, the cradle device is affixed to the skin of a patient. Next, a connection port is mounted on the cradle device, and a cannula provided in the connection port is punctured and indwelt into the patient. The pump main body, which stores and delivers the drug solution, is mounted on the cradle device. At this time, the connecting needle tube that delivers the drug solution in the pump main body is connected to the connection port (for example, refer to PTL 1) .

### Citation List

### Patent Literature

PTL 1: JP-T-2007-509661

### Summary of Invention

### Technical Problem

However, in the technology described in PTL 1, there is a concern that the pump main body is mounted on the cradle device diagonally in a predetermined orientation. When the pump main body is mounted diagonally, the connecting needle tube widens a rubber plug provided in the connection port, or the connecting needle tube or the connection port is deformed. As a result, there is a problem that a gap is generated between the connecting needle tube and the connection port, and drug solution leaks out.

In consideration of the above-described problem, the object is to provide a drug solution administration apparatus and a cradle device that can mount a pump main body in a correct mounting posture.

### Solution to Problem

In order to solve the above-described problem and achieve the object, the drug solution administration apparatus includes a pump main body, a cradle device, and a connection port. The pump main body has a drug solution storage section filled with a drug solution, a liquid delivery pipe connected to the drug solution storage section, and a housing that accommodates the drug solution storage section and the liquid delivery pipe. The cradle device has a placement surface portion on which the pump main body is placed, the pump main body being attachably and detachably mounted on the cradle device by sliding the pump main body in a first direction. The connection port has a port main body connected to the liquid delivery pipe and a cannula insertable into a living body, and is mounted on the placement surface portion. The housing includes a bottom surface portion placed on the placement surface portion, an upper surface portion that faces the bottom surface portion, and a side surface portion that is continuous from both end portions of the bottom surface portion and the upper surface portion in a second direction orthogonal to the first direction and connects the bottom surface portion and the upper surface portion to each other. The cradle device has side wall portions that rise in a third direction substantially orthogonal to the first direction and the second direction from both end portions of the placement surface portion in the second direction, and face the side surface portions.

The side wall portion has a posture correction section that is attached to or faces a corner portion where the upper surface portion and the side surface portion are connected to each other in the housing.

In the cradle device, a pump main body that administers a drug solution is attachably and detachably mounted. The cradle device includes : a placement surface portion of which a lower surface is affixed to a living body surface; a mounting section that is provided in the placement surface portion and capable of connecting a connection port having a cannula insertable into a living body; and a pair of side wall portions that rise from end portions of the placement surface portion in a direction opposite to the living body surface and face each other. The pair of side wall portions each have a protruding portion that further protrudes in the direction opposite to the living body surface. The protruding portion is curved such that an end portion of the protruding portion is at a position overlapping the placement surface portion, in a top view when the placement surface portion is viewed from a direction in which the pair of side wall portions rise.

### Advantageous Effect

According to the drug solution administration apparatus and the cradle device having the above-described configuration, the pump main body can be mounted in a correct mounting posture.

### Brief Description of Drawings

[Fig. 1] Fig. 1 is a perspective view showing a drug solution administration apparatus according to a first embodiment.
[Fig. 2] Fig. 2 is a perspective view showing a state before a pump main body is mounted on a cradle device in the drug solution administration apparatus according to the first embodiment.
[Fig. 3] Fig. 3 is a perspective view showing the pump main body of the drug solution administration apparatus according to the first embodiment.
[Fig. 4] Fig. 4 is a plan view showing an inner configuration of the pump main body of the drug solution administration apparatus according to the first embodiment.
[Fig. 5] Fig. 5 is a cross-sectional view showing a connection state of a connecting needle tube and a connection port of the drug solution administration apparatus according to the first embodiment.
[Fig. 6] Fig. 6 is a perspective view enlarging and showing the cradle device and the connection port in the drug solution administration apparatus according to the first embodiment.
[Fig. 7] Fig. 7 is a perspective view showing a state where the pump main body is mounted on the cradle device in the drug solution administration apparatus according to the first embodiment.
[Fig. 8] Fig. 8 is a side view showing a state where the pump main body is mounted on the cradle device in the drug solution administration apparatus according to the first embodiment.
[Fig. 9] Fig. 9 is a perspective view showing a state where the pump main body is mounted on the cradle device in the drug solution administration apparatus according to the first embodiment, and is a perspective view showing a state where a posture of the pump main body is corrected.
[Fig. 10] Fig. 10 is a perspective view showing a cradle device in a drug solution administration apparatus according to a second embodiment.
[Fig. 11] Fig. 11 is a perspective view showing a cradle device in a drug solution administration apparatus according to a third embodiment.
[Fig. 12] Fig. 12 is a perspective view showing a mounting opening of the cradle device in the drug solution administration apparatus according to the third embodiment.

### Description of Embodiments

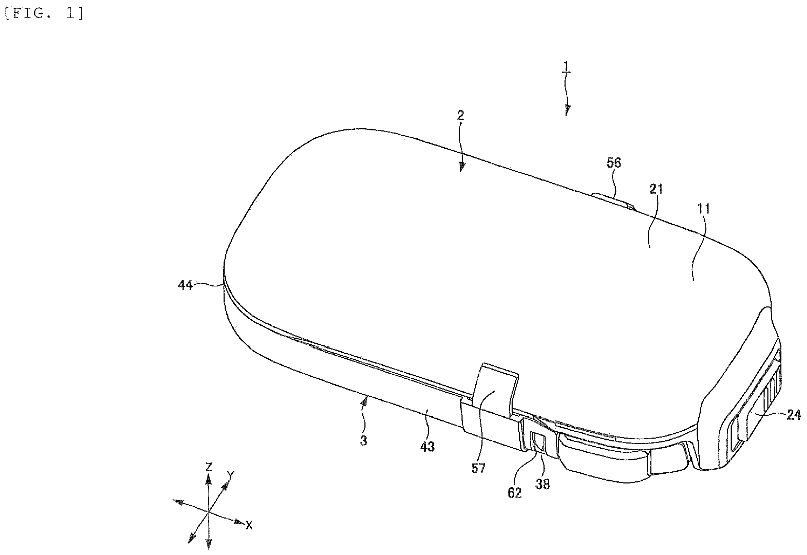

Hereinafter, embodiments of a drug solution administration apparatus and a cradle device will be described with reference to Figs. 1 to 12. Note that the common members in each drawing will be given the same reference numerals. The present invention is not limited to the following aspects.

### 1. First Embodiment

### 1-1. Configuration of Drug Solution Administration Apparatus

First, a configuration example of the drug solution administration apparatus according to a first embodiment (hereinafter referred to as "this example") will be described with reference to Figs. 1 to 5.

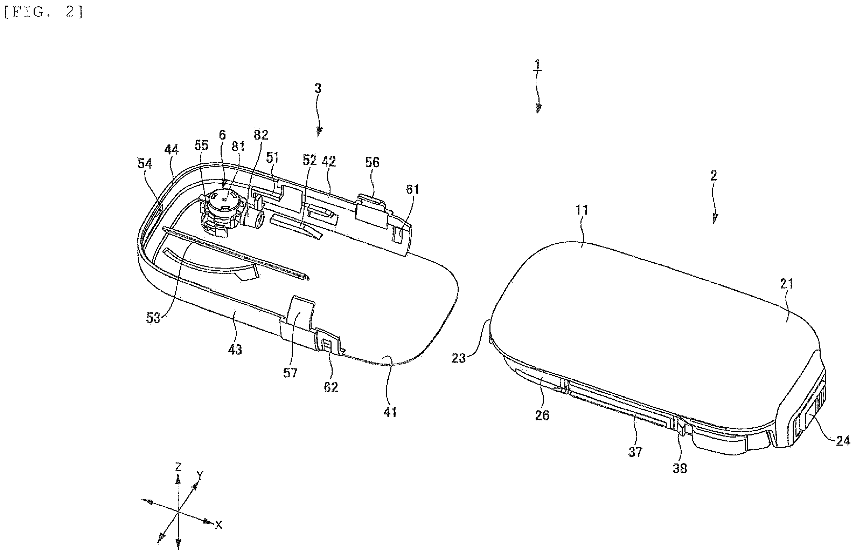

Fig. 1 is a perspective view showing a drug solution administration apparatus, and Fig. 2 is a perspective view showing a state before a pump main body is mounted on a cradle device in the drug solution administration apparatus.

A drug solution administration apparatus 1 shown in Fig. 1 is a portable insulin pump for continuous drug solution administration into the body of a patient, such as a patch type, tube type, or even other portable drug solution administration apparatuses. The drug solution administration apparatus 1 is affixed to a living body to be used. The drug solution administration apparatus 1 includes a pump main body 2, a cradle device 3 to which the pump main body 2 is mounted attachably and detachably, and a connection port 6 mounted on the cradle device 3.

### [Pump Main Body]

The configuration of the pump main body 2 is described with reference to Figs. 1 to 5.

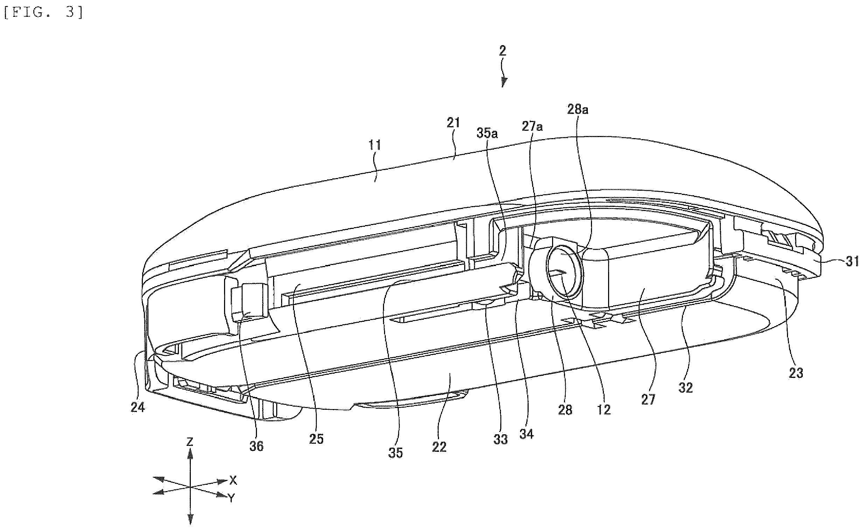

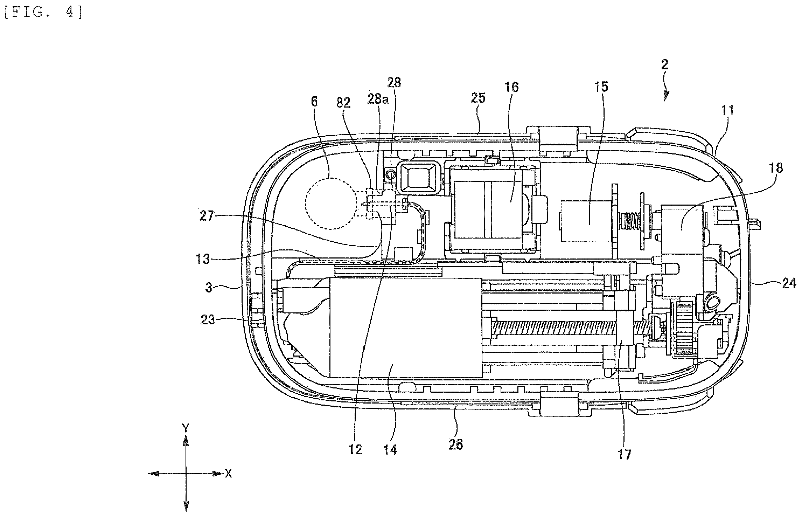

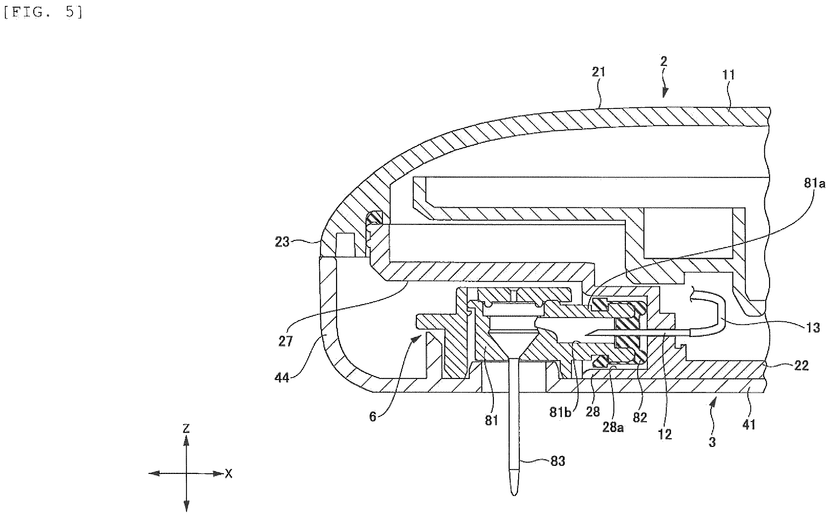

Fig. 3 is a perspective view of the pump main body 2, and Fig. 4 is a plan view showing the inner configuration of the pump main body 2. More specifically, Fig. 4 is a plan view showing the inner configuration of the pump main body 2 in a case where the upper surface portion 21 is not shown. Fig. 5 is a partial cross-sectional view showing a state where the pump main body 2 is mounted on the cradle device 3.

As shown in Figs. 1 to 3, the pump main body 2 includes a housing 11 and a connecting needle tube 12. As shown in Fig. 4, the housing 11 accommodates a liquid delivery pipe 13, a drug solution storage section 14, a driving section 15, a power supply section 16 that supplies power to the driving section 15, a plunger mechanism 17, and a transmission mechanism 18. The drug solution storage section 14 is filled with the drug solution to be administered. The drug solution storage section 14 is connected to the liquid delivery pipe 13. On the drug solution storage section 14, the plunger mechanism 17 that pushes out the drug solution is mounted.

The transmission mechanism 18 is connected to the plunger mechanism 17. The transmission mechanism 18 is connected to the driving section 15, and transmits the driving force of the driving section 15 to the plunger mechanism 17 via gears, and the like. As the driving force is transmitted, the plunger mechanism 17 is driven. As the plunger mechanism 17 is driven, the drug solution stored in the drug solution storage section 14 is delivered out toward the liquid delivery pipe 13.

The end portion on the opposite side of the drug solution storage section 14 side in the liquid delivery pipe 13 has the connecting needle tube 12. The drug solution that has passed through the liquid delivery pipe 13 is discharged from the connecting needle tube 12. As shown in Figs. 4 and 5, the connecting needle tube 12 is punctured into a rubber plug 82 provided in a connection section 81a of the connection port 6, which will be described later, when the pump main body 2 is mounted on the cradle device 3. Accordingly, the liquid delivery pipe 13 and the connection port 6 are connected via the connecting needle tube 12.

Hereinafter, the direction in which the pump main body 2 is mounted on the cradle device 3 (mounting direction) is referred to as a first direction X. The pump main body 2 is mounted by sliding in the direction from a first end to a second end of the cradle device 3. The direction orthogonal to the first direction X and orthogonal to the direction in which the pump main body 2 and the cradle device 3 overlap each other during the administration of the drug solution of the drug solution administration apparatus 1 is a second direction Y (width direction). In other words, the second direction Y is a direction that is substantially parallel to the living body surface when the drug solution administration apparatus 1 administers the drug solution. The direction in which the pump main body 2 and the cradle device 3 overlap each other, that is, the direction orthogonal to both the first direction X and the second direction Y is a third direction Z (up-down direction). Here, the third direction Z is a direction that is substantially orthogonal to the living body surface when the drug solution administration apparatus 1 administers the drug solution.

As shown in Figs. 2 and 3, the housing 11 is formed in a flat and substantially rectangular parallelepiped shape with curved corner portions. The housing 11 has the upper surface portion 21, a bottom surface portion 22, a front surface portion 23, a back surface portion 24, a first side surface portion 25, a second side surface portion 26, and a bottom surface accommodation section 27. The upper surface portion 21 and the bottom surface portion 22 are facing each other. The upper surface portion 21 is formed in a substantially rectangular shape with curved corner portions, in the top view. The upper surface portion 21 has the center portion bulging out in the third direction Z toward the opposite side of the bottom surface portion 22.

The front surface portion 23 is connected substantially perpendicularly to a second end portion of the upper surface portion 21 in the first direction X, and the back surface portion 24 is connected substantially perpendicularly to a first end portion of the upper surface portion 21 in the first direction X. The front surface portion 23 and the back surface portion 24 are facing each other. Furthermore, the first side surface portion 25 is connected substantially perpendicularly to one end portion of the upper surface portion 21 in the second direction Y, and the second side surface portion 26 is connected substantially perpendicularly to the other end portion of the upper surface portion 21 in the second direction Y.

As shown in Fig. 3, an engagement projection 31 is formed in the front surface portion 23. In a case where the front surface portion 23 is viewed from the second end in the first direction X toward the first end, the engagement projection 31 is formed on the other end portion side of the front surface portion 23 in the second direction Y. In other words, the engagement projection 31 is offset from the center of the second direction Y in housing 11 in the opposite direction to a connection opening 28 which will be described later. The engagement projection 31 protrudes from the front surface portion 23 toward the second end side in the first direction X. The engagement projection 31 is engaged with an engagement hole 54 provided in the cradle device 3 described below.

The bottom surface accommodation section 27 is formed in the corner portion of the bottom surface portion 22, the first side surface portion 25, and the front surface portion 23 in the housing 11. The bottom surface accommodation section 27 is a concave portion which is recessed with a predetermined area and a predetermined length (thickness) from the bottom surface portion 22 to the upper surface portion 21 along the third direction Z.

The tubular connection opening 28 is formed on a wall surface 27a on the first end portion side in the first direction X in the bottom surface accommodation section 27. The connection opening 28 has a tube hole 28a that protrudes from the wall surface 27a toward the second end portion in the first direction X. The connecting needle tube 12 is disposed in the tube hole 28a of the connection opening 28. The tube hole 28a protects the connecting needle tube 12 by protruding from the wall surface 27a to surround the connecting needle tube 12. The connecting needle tube 12 protrudes from the wall surface 27a in the tube hole 28a toward the second end portion side in the first direction X.

As shown in Figs. 4 and 5, when the pump main body 2 is mounted on the cradle device 3, the connection port 6 attached to the cradle device 3 is accommodated in the bottom surface accommodation section 27. Furthermore, the connection section 81a and the rubber plug 82 of the connection port 6 are inserted into the tube hole 28a of the connection opening 28.

As shown in Fig. 3, the bottom surface portion 22 has a sliding groove portion 32 and a detection groove portion 34. The sliding groove portion 32 and the detection groove portion 34 are concave portions recessed from the bottom surface portion 22 toward the upper surface portion 21 along the third direction Z. The sliding groove portion 32 is formed in the middle of the second direction Y in the bottom surface portion 22. The sliding groove portion 32 is formed continuously from the second end portion to the first end portion in the first direction X in the bottom surface portion 22.

The detection groove portion 34 is formed on one end portion side in the second direction Y in the bottom surface portion 22. The detection groove portion 34 extends from the wall surface 27a of the bottom surface accommodation section 27 toward the first end portion in the first direction X. In the detection groove portion 34, a mounting detection switch 33 is provided.

The mounting detection switch 33 is pressed by a detection rail 52 provided in the cradle device 3, which will be described later, when the pump main body 2 is mounted on the cradle device 3. Then, when the mounting detection switch 33 is pressed, the mounting information to the control unit, which is not shown, is output. Accordingly, the control unit detects that the pump main body 2 has been mounted on the cradle device 3.

As shown in Fig. 3, in the first side surface portion 25, a first guide groove portion 35 and a first engagement hook portion 36 are formed. The first engagement hook portion 36 is formed at the first end portion of the first direction X in the first side surface portion 25. The first engagement hook portion 36 is formed on the first end portion side of the first guide groove portion 35 in the first direction X. The first engagement hook portion 36 is attachably and detachably engaged with a first engagement receiving section 61 (refer to Fig. 2) of the cradle device 3, which will be described later.

The first guide groove portion 35 is formed in the middle of the third direction Z in the first side surface portion 25. The first guide groove portion 35 is a groove portion recessed from the first side surface portion 25 into the housing 11 along the second direction Y. The first guide groove portion 35 extends along the first direction X from the vicinity of the first engagement hook portion 36 in the first side surface portion 25.

An extension section 35a is formed at one end portion of the first guide groove portion 35, that is, on the bottom surface accommodation section 27 side. The extension section 35a protrudes from the wall surface 27a of the bottom surface accommodation section 27 toward the second end portion of the first direction X. The extension section 35a protrudes in the first direction X from the connecting needle tube 12 that protrudes from the wall surface 27a. Therefore, the second end portion of the first guide groove portion 35 in the first direction X extends to the second end portion side in the first direction X from the distal end of the connecting needle tube 12.

As shown in Fig. 2, in the second side surface portion 26, a second guide groove portion 37 and a second engagement hook portion 38 are formed. The second engagement hook portion 38 is formed at the first end portion of the first direction X in the second side surface portion 26. The second engagement hook portion 38 is attachably and detachably engaged with a second engagement receiving section 62 of the cradle device 3, which will be described later.

The second guide groove portion 37 is formed in the middle of the third direction Z in the second side surface portion 26. The second guide groove portion 37 is a groove portion recessed from the second side surface portion 26 into the housing 11 along the second direction Y. The second guide groove portion 37 extends from the second engagement hook portion 38 along the first direction X with a length that is substantially the same as the length in the first direction X in the first guide groove portion 35.

### [Cradle Device]

Next, the cradle device 3 will be described with reference to Figs. 1, 2, and 6.

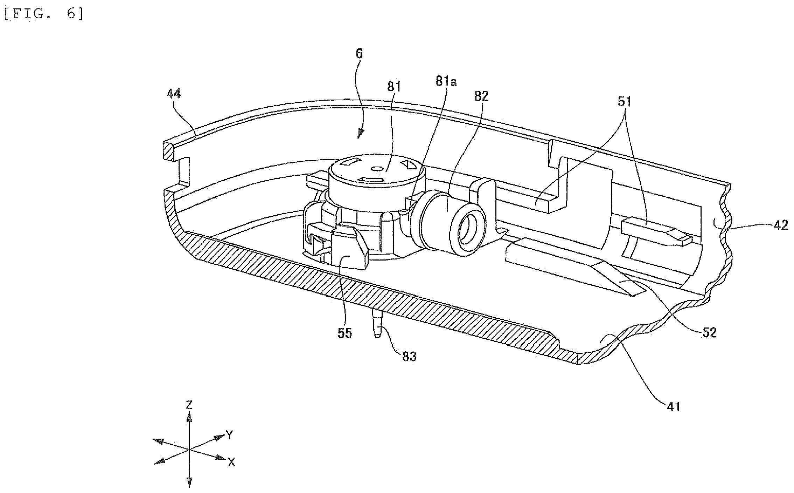

Fig. 6 shows a perspective view in which the cradle device 3 and the connection port 6 are enlarged and showed.

As shown in Fig. 1, the cradle device 3 is configured to be capable of carrying the pump main body 2 having the above-described configuration. As shown in Fig. 2, the cradle device 3 has a substantially flat plate-shaped placement surface portion 41, a first side wall portion 42, a second side wall portion 43, and a third side wall portion 44. The placement surface portion 41 is formed in a substantially rectangular shape with curved corner portions, in the top view. When the pump main body 2 is mounted on the cradle device 3, the bottom surface portion 22 of the housing 11 of the pump main body 2 is placed on the placement surface portion 41.

On one surface of the placement surface portion 41, the detection rail 52, a sliding rail 53, and a mounting section 55 are provided. The mounting section 55 is disposed at the second end portion of the placement surface portion 41 in the first direction X and is formed at one end portion in the second direction Y. As shown in Fig. 6, the connection port 6 is mounted on the mounting section 55. Furthermore, in the mounting section 55, an insertion hole (not shown) through which a cannula 83 of the connection port 6 (will be described later) is inserted is provided.

The detection rail 52 is formed at one end portion of the placement surface portion 41 in the second direction Y, that is, on the first end portion side of the mounting section 55 in the first direction X. The detection rail 52 is a projection portion that protrudes from one surface of the placement surface portion 41. The first end portion of the detection rail 52 in the first direction X has a gradual increase in thickness from the placement surface portion 41 as moving toward the second end portion side in the first direction X. The detection rail 52 extends for a predetermined length along the first direction X. When the pump main body 2 is mounted on the cradle device 3, the detection rail 52 enters the detection groove portion 34 provided on the pump main body 2. The detection rail 52 then presses the mounting detection switch 33.

The sliding rail 53 is formed in the middle of the second direction Y on one surface of the placement surface portion 41. The sliding rail 53 extends from the second end portion in the first direction X on one surface of the placement surface portion 41 toward the first end portion. When the pump main body 2 is mounted on the cradle device 3, the sliding rail 53 is slidably fitted with the sliding groove portion 32 provided in the bottom surface portion 22 of the pump main body 2.

At one end portion of the placement surface portion 41 in the second direction Y, the first side wall portion 42 is continuous substantially perpendicularly to the placement surface portion 41. At the other end portion of the placement surface portion 41 in the second direction Y, the second side wall portion 43 is continuous substantially perpendicularly to the placement surface portion 41. At the second end portion of the placement surface portion 41 in the first direction X, the third side wall portion 44 is continuous substantially perpendicularly to the placement surface portion 41. The first side wall portion 42 and the second side wall portion 43 face each other. Three of the four sides of the placement surface portion 41 is connected to the first side wall portion 42, the second side wall portion 43 and the third side wall portion 44. Therefore, the first side wall portion 42 and the second side wall portion 43, in which a plurality of side wall portions rise from the placement surface portion 41 in the opposite direction to the living body surface, have at least a part of the side parallel to the first direction X in the placement surface portion 41, more preferably, a length 50% or longer of a length of the sides parallel to each other in the first direction X. The first end of the placement surface portion 41 in the first direction X does not have a side wall portion that is continuous and substantially perpendicular to the placement surface portion 41. In other words, the first end of the placement surface portion 41 in the first direction X and a part of the first end portion are open. Accordingly, the pump main body 2 is inserted into the placement surface portion 41 from the first end side in the first direction X.

In a state where the pump main body 2 is mounted on the cradle device 3, the first side wall portion 42 faces the first side surface portion 25 of the housing 11 in the pump main body 2, and the second side wall portion 43 faces the second side surface portion 26. The third side wall portion 44 faces the front surface portion 23 of the housing 11. At least a part of the pump main body 2 is surrounded by the first side wall portion 42, the second side wall portion 43, and the third side wall portion 44, and is accommodated inside the cradle device 3.

As shown in Fig. 2, in the third side wall portion 44, the engagement hole 54 is open. When the pump main body 2 is mounted on the cradle device 3, the engagement hole 54 is engaged with the engagement projection 31 of the pump main body 2.

In the first side wall portion 42, a first guide rail 51, a first posture correction section 56, and the first engagement receiving section 61 are formed. The first engagement receiving section 61 is formed at the first end portion of the first side wall portion 42 in the first direction X. The first engagement receiving section 61 is an opening portion with the first side wall portion 42 cut out in a substantially rectangular shape. When the pump main body 2 is mounted on the cradle device 3, the first engagement hook portion 36 is attachably and detachably engaged with the first engagement receiving section 61.

As shown in Fig. 6, the first guide rail 51 protrudes from one surface facing the second side wall portion 43 in the first side wall portion 42 toward the other end portion in the second direction Y. The first guide rail 51 is a projection portion formed parallel to the first direction X from the second end portion of the first side wall portion 42 in the first direction X. The second end portion of the first guide rail 51 in the first direction X extends to the position of the connection port 6 attached to the mounting section 55. More specifically, the first guide rail 51 extends beyond the second end portion of the connection section 81a.

When the pump main body 2 is mounted on the cradle device 3, the first guide rail 51 is engaged with the first guide groove portion 35 provided on the first side surface portion 25 of the pump main body 2. Accordingly, the pump main body 2 is guided in the mounting direction.

As shown in Figs. 1 and 2, the first posture correction section 56 is a plate-like protruding portion extending from the first side wall portion 42. The first posture correction section 56 extends further upward in the third direction Z from the first side wall portion 42. In other words, the first posture correction section 56 extends from the first side wall portion 42 in the direction opposite to the placement surface portion 41. The first posture correction section 56 is formed on the first end portion side of the first guide rail 51 in the first direction X, in the first side wall portion 42. In this example, the first posture correction section 56 is formed at the first end portion of the first side wall portion 42 in the first direction X.

The first posture correction section 56 protrudes in the third direction Z from one end portion of the first side wall portion 42 in the third direction Z and is curved toward the other end portion in the second direction Y. In other words, the first posture correction section 56 is formed in a shape corresponding to the shape of the connection section (corner portion) between the upper surface portion 21 and the first side surface portion 25 in the housing 11. The distal end and/or the distal portion of the first posture correction section 56 is positioned above the placement surface portion 41 in the third direction Z in a side view from the first direction X of the cradle device 3. In a plan view from the third direction Z, the first posture correction section 56 covers at least a part of the upper surface portion 21.

As shown in Figs. 1 and 2, the second side wall portion 43 has a second guide rail (not shown), a second posture correction section 57, and the second engagement receiving section 62. The second engagement receiving section 62 is formed at the first end portion of the second side wall portion 43 in the first direction X. The second engagement receiving section 62 is an opening portion with the second side wall portion 43 cut out in a substantially rectangular shape. When the pump main body 2 is mounted on the cradle device 3, the second engagement hook portion 38 is attachably and detachably engaged with the second engagement receiving section 62.

The second guide rail protrudes from one surface facing the first side wall portion 42 in the second side wall portion 43 toward one end portion in the second direction Y. The second guide rail is a projection portion formed parallel to the direction toward the first end from the second end portion of the second side wall portion 43 in the first direction X. When the pump main body 2 is mounted on the cradle device 3, the second guide rail is engaged with the second guide groove portion 37 provided on the second side surface portion 26 of the pump main body 2. Accordingly, the pump main body 2 is guided in the mounting direction.

The second posture correction section 57 is a plate-like protruding portion extending from the second side wall portion 43. The second posture correction section 57 extends further upward in the third direction Z from the second side wall portion 43. In other words, the second posture correction section 57 extends from the second side wall portion 43 in the direction opposite to the placement surface portion 41. The second posture correction section 57 is formed on the first end portion side of the second guide rail in the first direction X, in the second side wall portion 43. The second posture correction section 57 is formed at a position facing the first posture correction section 56 formed in the first side wall portion 42. In this example, the second posture correction section 57 is formed at the first end portion of the second side wall portion 43 in the first direction X.

The second posture correction section 57 protrudes in the third direction Z from one end portion of the second side wall portion 43 in the third direction Z and is curved toward one end portion in the second direction Y. In other words, the second posture correction section 57 is formed in a shape corresponding to the shape of the connection section (corner portion) between the upper surface portion 21 and the second side surface portion 26 in the housing 11. The distal end and/or the distal portion of the second posture correction section 57 is positioned above the placement surface portion 41 in the third direction Z in a side view from the first direction X of the cradle device 3. In a plan view from the third direction Z, the second posture correction section 57 covers at least a part of the upper surface portion 21.

When the pump main body 2 is to be mounted on the cradle device 3, the first posture correction section 56 is attached to the corner portion where the first side surface portion 25 and the upper surface portion 21 are connected to each other in the housing 11 of the pump main body 2. The second posture correction section 57 is attached to the second side surface portion 26 in housing 11 and the corner portion of the upper surface portion 21. The first posture correction section 56 and the second posture correction section 57 correct the posture of the pump main body 2 when the pump main body 2 is mounted.

Note that the first posture correction section 56 and the second posture correction section 57 are disposed at least on the first end portion side of the connection port 6 mounted on the mounting section 55 in the first direction X, that is, on the upstream side of the pump main body 2 in the mounting direction.

In the cradle device 3, an affixing sheet that is affixed to the skin of the patient is provided. The affixing sheet is attached to the other surface opposite to one surface of the placement surface portion 41 of the cradle device 3. The affixing sheet has an opening portion (not shown) through which the cannula 83 of the connection port 6 (which will be described below) penetrates.

The affixing sheet is made of a flexible member, and an adhesive layer affixed to the skin of the patient is formed on the surface opposite to the placement surface portion 41. In a state before being affixed to the skin of the patient, the adhesive layer of the affixing sheet is covered with a separation paper.

### [Connection Port]

Next, the connection port 6 will be described with reference to Figs. 5 and 6.

As shown in Figs. 5 and 6, the connection port 6 has a port main body 81 and the cannula 83. The cannula 83 is held in the port main body 81. In the port main body 81, the tubular connection section 81a is formed. A tube hole 81b of the connection section 81a and the cannula 83 communicate with each other. The rubber plug 82 is mounted on the distal portion in the connection section 81a.

When the connection port 6 is mounted on the mounting section 55 of the cradle device 3, the cannula 83 penetrates the placement surface portion 41 along the third direction Z and protrudes from the other surface of the placement surface portion 41. The cannula 83 is then punctured and indwelt into the living body.

The connection section 81a of the port main body 81 faces the first end portion side of the first direction X, that is, the upstream side of the mounting direction. The rubber plug 82 is disposed on the first end portion side of the connection section 81a in the first direction X. When the pump main body 2 is mounted on the cradle device 3, the rubber plug 82 is punctured by the connecting needle tube 12 of the pump main body 2. Accordingly, the port main body 81 is connected to the liquid delivery pipe 13 of the pump main body 2, and the liquid delivery pipe 13 communicates with the cannula 83. The drug solution stored in the drug solution storage section 14 of the pump main body 2 is delivered to the connection port 6 via the liquid delivery pipe 13 and administered to the patient through the cannula 83 when the driving section 15 is driven.

### 1-2. Mounting Operation Example of Pump Main Body

Next, an example of the mounting operation of mounting the pump main body 2 on the cradle device 3 in the drug solution administration apparatus 1 having the above-described configuration will be described with reference to Figs. 1, 2, and 7 to 9.

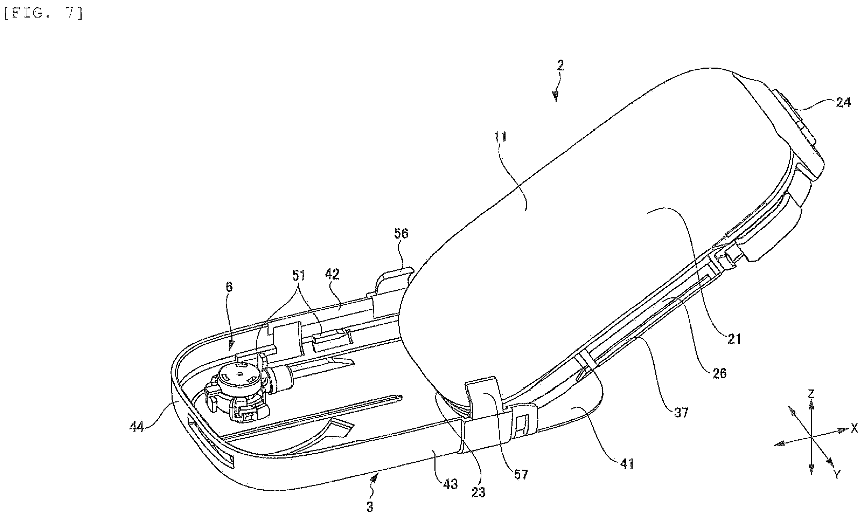

Figs. 7 to 9 are views showing a state where the pump main body 2 is mounted on the cradle device 3.

First, the separation paper of the affixing sheet in the cradle device 3 is peeled off, and the affixing sheet is affixed to the skin of the patient. Accordingly, the cradle device 3 is attached to the skin of the patient via the affixing sheet. Then, as shown in Fig. 2, the connection port 6 is mounted on the mounting section 55 of the cradle device 3, and the cannula 83 is punctured and indwelt in the patient. Next, as shown in Fig. 2, the pump main body 2 is inserted from the first end portion side of the cradle device 3 in the first direction X.

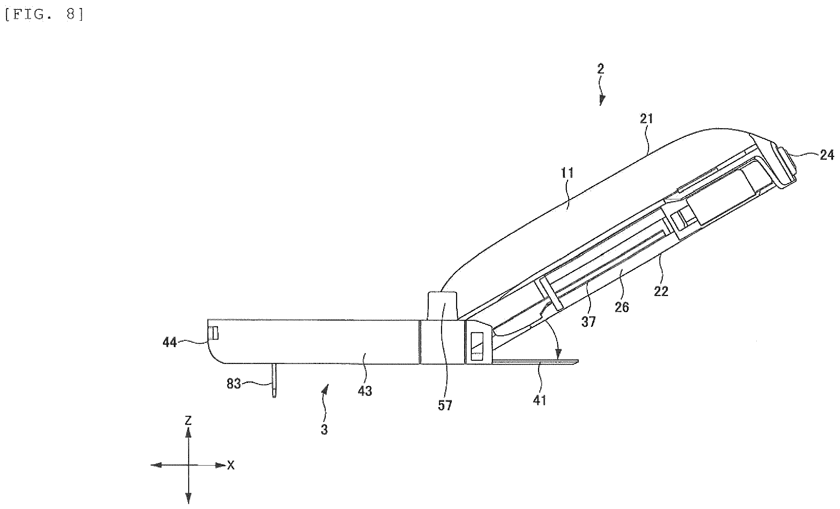

Here, as shown in Figs. 7 and 8, an example in which the mounting is performed in a state where the pump main body 2 is tilted against the cradle device 3, that is, a state where the bottom surface portion 22 of the pump main body 2 is not parallel or substantially parallel to the placement surface portion 41. In this state, the bottom surface portion 22 is inclined to one surface of the placement surface portion 41 in the first direction X and/or the second direction Y.

In a state shown in Figs. 7 and 8, when the pump main body 2 is moved toward the second end portion in the first direction X, the first posture correction section 56 and the second posture correction section 57 which are provided on the cradle device 3 are attached to the upper surface portion 21 of the pump main body 2. In this state, when the pump main body 2 is moved further toward the second end portion in the first direction X, the pump main body 2 is pressured in the direction toward the cradle device 3 by the first posture correction section 56 and the second posture correction section 57.

The corner portion of the housing 11 of the pump main body 2 is formed in a rounded and curved shape. Therefore, even when the pump main body 2 is moved to the second end side in the first direction X while the pump main body 2 is tilted against the cradle device 3, the pump main body 2 moves rotationally in the direction in which the bottom surface portion 22 approaches the placement surface portion 41 due to interference between the first and second posture correction sections 56 and 57 and the corner portion of the housing 11, respectively.

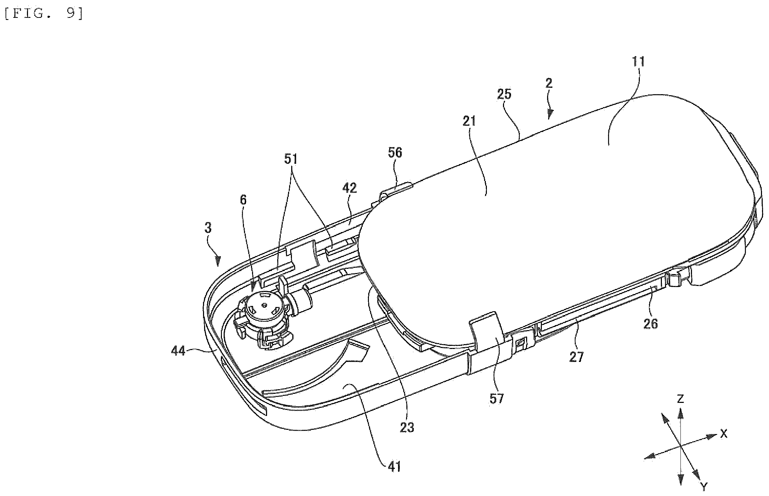

Accordingly, as shown in Fig. 9, the tilt of the pump main body 2 is corrected, and the bottom surface portion 22 of the pump main body 2 is parallel to the second direction Y and the first direction X. As a result, accordingly, that the first guide groove portion 35 and the second guide groove portion 37, which are provided on the pump main body 2, can be reliably engaged with the first guide rail 51 and the second guide rail.

Next, the pump main body 2 is made slide on the placement surface portion 41 toward the second end portion in the first direction X along the first guide rail 51 and the second guide rail. The first posture correction section 56 and the second posture correction section 57 are facing the corner portion of the upper surface portion 21 and the first side surface portion 25 and the corner portion of the upper surface portion 21 and the second side surface portion 26 in the housing 11. Therefore, when sliding the pump main body 2, the pump main body 2 lifts up from the placement surface portion 41 of the cradle device 3 and is to leave the placement surface portion 41, the pump main body 2 is attached to the first posture correction section 56 and the second posture correction section 57.

Accordingly, the posture of the pump main body 2 when sliding the pump main body 2 into the cradle device 3 can also be corrected to the correct posture by the first posture correction section 56 and the second posture correction section 57. As a result, the pump main body 2 can be slid straight along the placement surface portion 41 of the cradle device 3. Furthermore, the first posture correction section 56 and the second posture correction section 57 can prevent the pump main body 2 from being lifted up, and thus, the vibration generated in the pump main body 2 is suppressed after the pump main body 2 is mounted on the cradle device 3 .

As shown in Fig. 5, the rubber plug 82 and the connection section 81a of the connection port 6 are inserted into the tube hole 28a of the connection opening 28. Then, the connecting needle tube 12 of the pump main body 2 is punctured into the rubber plug 82 of the connection port 6, and the connection port 6 and the liquid delivery pipe 13 communicate with each other. Accordingly, the drug solution storage section 14 of the pump main body 2 and the cannula 83 of the connection port 6 are connected to each other via the liquid delivery pipe 13, the connecting needle tube 12 and the port main body 81.

Note that, during the mounting operation of the pump main body 2, the posture of the pump main body 2 with respect to the cradle device 3 is corrected by the first posture correction section 56 and the second posture correction section 57, and thus, the connecting needle tube 12 can be punctured and connected to the rubber plug 82 at the correct angle and direction. Accordingly, it is possible to prevent the connecting needle tube 12 from widening the rubber plug 82, or the connecting needle tube 12 or the connection port 6 from deforming. As a result, a gap is generated between the connecting needle tube 12 and the rubber plug 82, the drug solution is prevented from leaking out.

Furthermore, when the pump main body 2 is moved toward the second end portion side in the first direction X, the engagement projection 31 is engaged with the engagement hole 54 of the cradle device 3. Then, the first engagement hook portion 36 is engaged with the first engagement receiving section 61, and the second engagement hook portion 38 is engaged with the second engagement receiving section 62. Accordingly, the pump main body 2 mounted on the cradle device 3 can be prevented from falling out of the cradle device 3.

The detection rail 52 of the cradle device 3 slides through the detection groove portion 34 of the pump main body 2 and presses the mounting detection switch 33. Accordingly, the control unit (not shown) provided in the pump main body 2 detects that the pump main body 2 has been mounted on the cradle device 3. As a result, the mounting operation of the pump main body 2 on the cradle device 3 is completed.

According to the drug solution administration apparatus 1 in this example, the first posture correction section 56 and the second posture correction section 57 correct the posture of the pump main body 2 while the pump main body 2 is being mounted on the cradle device 3. By mounting the pump main body 2 on the cradle device 3 in an appropriate posture, it is possible to prevent the mounting detection switch 33 from being pressed by the detection rail 52 in an inappropriate mounting state where the pump main body 2 is tilted against the cradle device 3, and to prevent false detection from occurring. As a result, the mounting state of the pump main body 2 on the cradle device 3 is reliably detected by the mounting detection switch 33.

In a side view from the first direction X, the distal portion of the first posture correction section 56 and the distal portion of the second posture correction section 57 face the placement surface portion 41 in the third direction Z. Therefore, in a case where the pump main body 2 is to be mounted on the placement surface portion 41 of the cradle device 3 from above in the third direction Z, the first posture correction section 56 and the second posture correction section 57 are attached to the lower surface of the pump main body 2, and thus, the mounting operation of the pump main body 2 is prevented. Accordingly, the first posture correction section 56 and the second posture correction section 57 can prevent the pump main body 2 from being mounted in the wrong direction.

Note that, in the above-described embodiment, an example was described in which the projecting guide rail 51 is provided on the cradle device 3 side and the first guide groove portion 35 and the second guide groove portion 37 which are engaged with the first guide rail 51 and the second guide rail are provided on the pump main body 2 side, but the present invention is not limited thereto. For example, a guide groove portion may be provided on the cradle device 3 side, and a guide rail which is engaged with the guide groove portion may be provided on the pump main body 2 side.

### 2. Second Embodiment

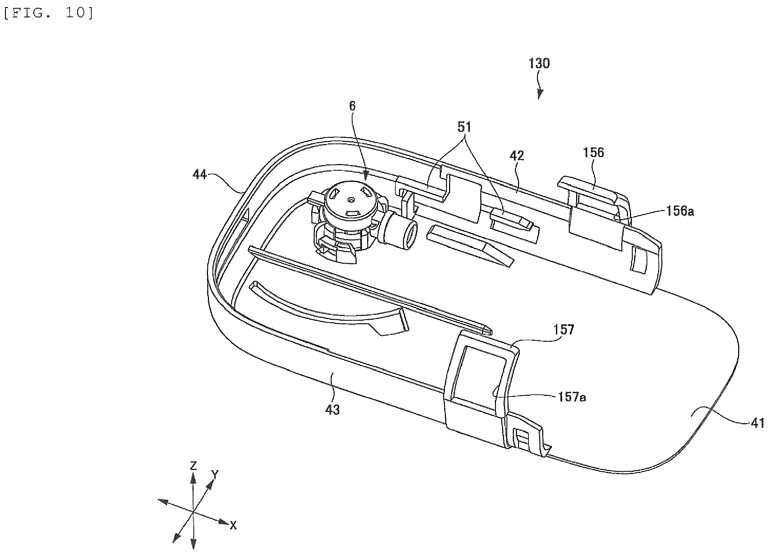

Next, the cradle device according to the second embodiment will be described with reference to Fig. 10.

Fig. 10 is a perspective view showing a cradle device according to a second embodiment.

The difference between the cradle device in the second embodiment and the cradle device 3 in the first embodiment is the configuration of the posture correction section. Therefore, the configuration of the posture correction section will be described here, parts that are common to the cradle device according to the first embodiment will be given the same reference numerals, and the duplicated description thereof will be omitted.

As shown in Fig. 10, a cradle device 130 on which the connection port 6 is mounted includes the placement surface portion 41, the first side wall portion 42, the second side wall portion 43, and the third side wall portion 44. A first guide rail 51 is formed in the first side wall portion 42, and a second guide rail (not shown) is formed in the second side wall portion 43. A first posture correction section 156 is formed in the first side wall portion 42, and a second posture correction section 157 is formed in the second side wall portion 43.

The first posture correction section 156 and the second posture correction section 157 are formed on the first end portion side of the first guide rail 51 and the second guide rail in the first direction X, in the first side wall portion 42 and the second side wall portion 43. The first posture correction section 156 and the second posture correction section 157 have rectangular opening portions 156a and 157a, respectively. The first posture correction section 156 and the second posture correction section 157 are provided with elasticity.

The first posture correction section 156 and the second posture correction section 157 protrude such that the distal portions thereof are positioned up to the vicinity of the middle in the second direction Y. The length of the first posture correction section 156 and the second posture correction section 157 in the first direction X is set to be longer than the length of the first posture correction section 56 and the second posture correction section 57 in the first direction X according to the first embodiment.

Therefore, the first posture correction section 156 and the second posture correction section 157 are formed larger than the first posture correction section 56 and the second posture correction section 57 according to the first embodiment. When the pump main body 2 is mounted on the cradle device 130, the first posture correction section 156 covers a part of the upper surface portion 21 from the first side surface portion 25 in the pump main body 2, and the second posture correction section 157 covers a part of the upper surface portion 21 from the second side surface portion 26.

Accordingly, the first posture correction section 156 and the second posture correction section 157 can not only reliably correct the posture of the pump main body 2 when the pump main body 2 is mounted, but also increase the holding force to hold the pump main body 2.

The other configurations are the same as those of the cradle device 3 according to the first embodiment, and thus, the description thereof will be omitted. The cradle device 130 having such a configuration can also have the same effect as that of the cradle device 3 according to the above-described first embodiment.

### 3. Third Embodiment

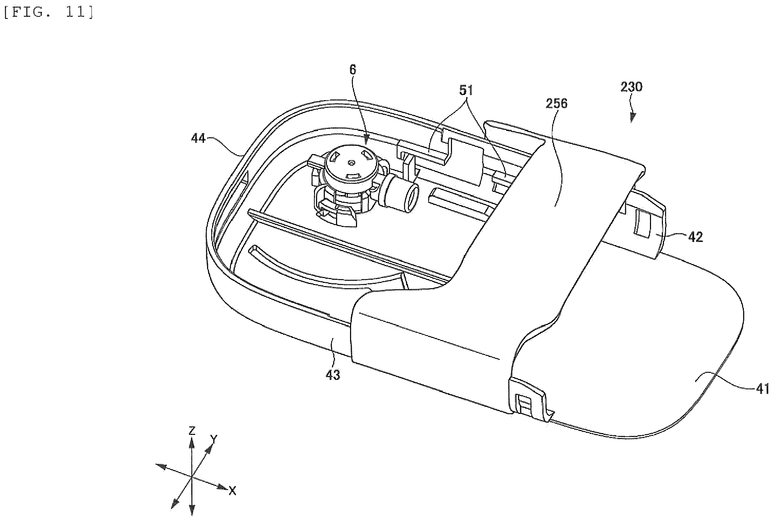

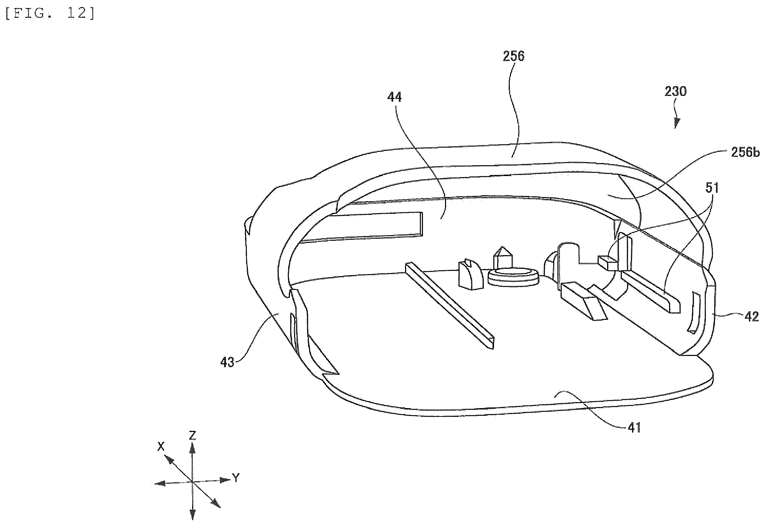

Next, the cradle device for the third embodiment will be described with reference to Figs. 11 and 12.

Figs. 11 and 12 are perspective views showing a cradle device according to a third embodiment.

The difference between the cradle device in the third embodiment and the cradle device 3 in the first embodiment is the configuration of the posture correction section. Therefore, the configuration of the posture correction section will be described here, parts that are common to the cradle device according to the first embodiment will be given the same reference numerals, and the duplicated description thereof will be omitted.

As shown in Figs. 11 and 12, a cradle device 230 on which the connection port 6 is mounted includes the placement surface portion 41, the first side wall portion 42, the second side wall portion 43, and the third side wall portion 44. The first guide rail 51 is formed in the first side wall portion 42, and the second guide rail (not shown) is formed in the second side wall portion 43. A posture correction section 256 is formed in the cradle device 230.

The posture correction section 256 is formed on the first end portion side of the first guide rail 51 and the second guide rail in the first direction X, in the first side wall portion 42 and the second side wall portion 43. The posture correction section 256 is formed to connect one end portion of the first side wall portion 42 and the second side wall portion 43 in the third direction Z. Therefore, the posture correction section 256 is provided opposite to the placement surface portion 41. The posture correction section 256 covers at least a part of each of the upper surface portion 21, the first side surface portion 25, and the second side surface portion 26 of the pump main body 2 that has been completely mounted on the cradle device 230.

As shown in Fig. 12, in the cradle device 230, an insertion opening 256b surrounded by the placement surface portion 41, the first side wall portion 42, the second side wall portion 43, and the posture correction section 256, is formed. The pump main body 2 is inserted from the first end portion side of the insertion opening 256b in the first direction X. Accordingly, the posture correction section 256 can not only correct the posture of the pump main body 2 when the pump main body 2 is mounted, but also restrict the mounting direction of the pump main body 2 onto the cradle device 230.

The other configurations are the same as those of the cradle device 3 according to the first embodiment, and thus, the description thereof will be omitted. The cradle device 230 having such a configuration can also have the same effect as that of the cradle device 3 according to the above-described first embodiment.

In the cradle devices 130 and 230 according to the second and third embodiments described above, the posture correction sections 156, 157, and 256 are larger than the posture correction sections 56 and 57 according to the first embodiment. Accordingly, in the cradle devices 130 and 230 according to the second and third embodiments, it is possible to reliably correct the posture of the pump main body 2 when the pump main body 2 is mounted.

In contrast, in the cradle device 3 according to the first embodiment, the posture correction sections 56 and 57 are smaller than the posture correction sections 156, 157, and 256 according to the second and third embodiments, and are attached to the corner portion of the pump main body 2. In other words, the posture correction sections 56 and 57 according to the first embodiment can reduce the protrusion amount from the housing 11 of the pump main body 2 than that of the posture correction sections 156, 157, and 256 according to the second and third embodiments. Accordingly, when using the drug solution administration apparatus 1, it is possible to suppress a case where the clothing and the like of the patient get caught in the posture correction sections 56 and 57 .

Above, embodiments of the present invention, including the operation effects have been described. However, the drug solution administration apparatus of the present invention is not limited to the above-described embodiments, and various variations can be implemented within the scope not departing from the gist of the present invention described in the claims.

In the above-described embodiments, an example has been described in which an insulin pump that administers insulin is applied as a drug solution administration apparatus, but the present invention is not limited thereto. Various other drug solutions, such as analgesics, anti-cancer drugs, HIV drugs, iron chelators, pulmonary hypertension drugs, and the like, may be used as drug solution to be administered. Not being limited to drug solution administration apparatuses that are affixed to the skin of the patient, the present invention can also be applied to other apparatuses that are used in combination with cradle devices that can be attached to the clothing or belongings of the patient. For example, body fluid component measurement apparatuses used for monitoring blood glucose levels, and the like, and patient information collection devices that collect information such as heart rate or number of steps, are exemplified, and it is needless to say that these apparatuses can be utilized in a multifaceted manner in the member communication function.

Note that, in the present specification, although words such as "parallel" and "orthogonal" were used, these do not mean only strict "parallel" and "orthogonal", but may also mean a state of being "substantially parallel" or "substantially orthogonal" including "parallel" and "orthogonal" and within the range of capable of performing the functions.

### Reference Signs List

- 1: DRUG SOLUTION ADMINISTRATION APPARATUS
- 2: PUMP MAIN BODY
- 3 130 230: CRADLE DEVICE
- 6: CONNECTION PORT
- 11: HOUSING
- 12: CONNECTING NEEDLE TUBE
- 13: LIQUID DELIVERY PIPE
- 14: DRUG SOLUTION STORAGE SECTION
- 15: DRIVING SECTION
- 16: POWER SUPPLY SECTION
- 17: PLUNGER MECHANISM
- 18: TRANSMISSION MECHANISM
- 21: UPPER SURFACE PORTION
- 22: BOTTOM SURFACE PORTION
- 23: FRONT SURFACE PORTION
- 24: BACK SURFACE PORTION
- 25: FIRST SIDE SURFACE PORTION
- 26: SECOND SIDE SURFACE PORTION
- 27: BOTTOM SURFACE ACCOMMODATION SECTION
- 27a: WALL SURFACE
- 28: CONNECTION OPENING
- 28a: TUBE HOLE
- 31: ENGAGEMENT PROJECTION
- 32: SLIDING GROOVE PORTION
- 33: MOUNTING DETECTION SWITCH
- 34: DETECTION GROOVE PORTION
- 35: FIRST GUIDE GROOVE PORTION
- 35a: EXTENSION SECTION
- 36: FIRST ENGAGEMENT HOOK PORTION
- 37: SECOND GUIDE GROOVE PORTION
- 38: SECOND ENGAGEMENT HOOK PORTION
- 41: PLACEMENT SURFACE PORTION
- 42: FIRST SIDE WALL PORTION
- 43: SECOND SIDE WALL PORTION
- 44: THIRD SIDE WALL PORTION
- 51: FIRST GUIDE RAIL
- 52: DETECTION RAIL
- 53: SLIDING RAIL
- 54: ENGAGEMENT HOLE
- 55: MOUNTING SECTION
- 56 57 156 157 256: POSTURE CORRECTION SECTION (PROTRUDING PORTION)
- 81: PORT MAIN BODY
- 81A: CONNECTION SECTION
- 82: RUBBER PLUG
- 83: CANNULA
- 256b: INSERTION OPENING
- X: FIRST DIRECTION (MOUNTING DIRECTION)
- Y: SECOND DIRECTION (WIDTH DIRECTION)
- Z: THIRD DIRECTION (UP-DOWN DIRECTION)

## Claims

1. A drug solution administration apparatus comprising:
a pump main body having a drug solution storage section filled with a drug solution, a liquid delivery pipe connected to the drug solution storage section, and a housing that accommodates the drug solution storage section and the liquid delivery pipe;
a cradle device that has a placement surface portion on which the pump main body is placed, the pump main body being attachably and detachably mounted on the cradle device by sliding the pump main body in a first direction; and
a connection port that has a port main body connected to the liquid delivery pipe and a cannula insertable into a living body, and is mounted on the placement surface portion, wherein
the housing includes
a bottom surface portion placed on the placement surface portion,
an upper surface portion that faces the bottom surface portion, and
a side surface portion that is continuous from both end portions of the bottom surface portion and the upper surface portion in a second direction orthogonal to the first direction and connects the bottom surface portion and the upper surface portion to each other,
the cradle device has side wall portions that rise in a third direction substantially orthogonal to the first direction and the second direction from both end portions of the placement surface portion in the second direction, and face the side surface portions, and
the side wall portion has a posture correction section that is attached to or faces a corner portion where the upper surface portion and the side surface portion are connected to each other in the housing.

2. The drug solution administration apparatus according to claim 1, wherein
the posture correction section is disposed on an upstream side in the first direction from the connection port mounted on the cradle device.

3. The drug solution administration apparatus according to claim 2, wherein
one of the side wall portion and the housing is provided with a protruding guide rail formed parallel to the first direction,
the remaining other one of the side wall portion and the housing is provided with a guide groove portion that is slidably engaged with the guide rail, and
the posture correction section is disposed on an upstream side in the first direction from the guide rail or the guide groove portion which is provided on the side wall portion.

4. The drug solution administration apparatus according to any one of claims 1 to 3, wherein
the posture correction section protrudes in the third direction and the second direction from an end portion of the side wall portion on an opposite side of the placement surface portion, and faces or is attached to the upper surface portion of the housing.

5. The drug solution administration apparatus according to any one of claims 1 to 4, wherein
the posture correction section is a plate-like member attached to the corner portion in the housing.

6. The drug solution administration apparatus according to any one of claims 1 to 5, wherein
the posture correction section is formed according to a shape of the corner portion in the housing.

7. The drug solution administration apparatus according to any one of claims 1 to 6, wherein
the corner portion in the housing is formed in a curved shape.

8. A cradle device on which a pump main body that administers a drug solution is attachably and detachably mounted, the device comprising:
a placement surface portion of which a lower surface is affixed to a living body surface;
a mounting section that is provided in the placement surface portion and capable of connecting a connection port having a cannula insertable into a living body; and
a pair of side wall portions that rise from end portions of the placement surface portion in a direction opposite to the living body surface and face each other, wherein
the pair of side wall portions each have a protruding portion that further protrudes in the direction opposite to the living body surface, and
the protruding portion is curved such that an end portion of the protruding portion is at a position overlapping the placement surface portion, in a top view when the placement surface portion is viewed from a direction in which the pair of side wall portions rise.
